# EUROPEAN PATENT SPECIFICATION

(11) **EP 0 563 142 B1**
(45) Date of publication and mention of the grant of the patent: **13.12.1995**
(21) Application number: 92901381.1
(22) Date of filing: 05.12.1991
(51) Int. Cl.: C10G 32/00, C12S 1/02, C12N 1/20

(54) **Use of a biocatalyst for the reduction of petroleum viscosity**
Verwendung eines Biokatalysators zur Viskositätsminderung von Erdöl
Utilisation d'un biocatalyseur pour réduire la viscosité du pétrole

(30) Priority: 21.12.1990 US 631642
(43) Date of publication of application: 06.10.1993
(73) Proprietor: ENERGY BIOSYSTEMS CORPORATION, The Woodlands Texas 77381 (US)
(72) Inventor: MONTICELLO, Daniel, J., The Woodlands, TX 77381 (US); HANEY, William, H, III, Boston, MA 02115 (US)
(74) Representative: Harvey, David Gareth
(86) International application number: PCT/US91/09216
(87) International publication number: WO 92/11343

(56) References cited:
- EP-A- 0 401 922
- EP-A- 0 445 896
- US-A- 3 069 325
- US-A- 4 632 906

## Description

### BACKGROUND

The high viscosity of many crude oils is a factor contributing to the underutilization of these valuable resources. High viscosity greatly complicates, and may even defeat, the extraction of such resources from the Earth, such as in the case of Cerro Negro crude oil from Venezuela. In less extreme cases, high viscosity significantly hampers not only the extraction, but also the pumping, transportation, refining and handling of petroleum liquids, including crude oil. Because of this, the petroleum industry has long recognized the need for a method of accomplishing a safe, economical, and effective reduction in the viscosity of these valuable fuel resources.

One refining process which results in the reduction of viscosity in petroleum liquids is hydrodesulfurization (HDS), which is the treatment of petroleum with hydrogen gas under conditions of high temperature and pressure. Reduced viscosity is partly the result of the increased temperature to which the liquid is exposed, and partly due to the destruction of complex aromatic hydrocarbons, which are valuable sources of fuel energy upon combustion. Condensed ring aromatic molecules, which may include heterocyclic ring moieties, form a significant proportion of such aromatic molecules. Heterocyclic rings are those in which the aromatic ring contains one or more non-carbon substituents; an example of a sulfur-bearing heterocycle found in petroleum is dibenzothiophene. Although these molecules can be nonspecifically destroyed by HDS, they are refractory to this refining process. Due to the extreme conditions involved, HDS is useful only at a limited number of steps in the refining of petroleum, and is not a generally accepted method for reducing the viscosity of crude oil.

Microorganisms which are capable of accomplishing the objectives of HDS without the need to expose the petroleum being treated to extreme conditions of temperature and pressure have long been sought. One example of a microbial culture said to fulfill the desired criteria is reported in European Patent Application No. 0,401,922, dated 12 December 1990 (AGIP PETROLI S.p.A. and ENICHEM ANIC S.p.A., Applicants; Pifferi *et al*., invs.). Pifferi *et al*. claim a mixed culture comprising prevalently obligate anaerobic *Desulfovibrio desulfuricans* sp. microorganisms, together with *Clostridium* sp. sporigenic anaerobes and at least one facultative anaerobic coccus. Treatment of petroleum with this mixed culture is said to produce a high degree of desulfurization of the substrate, with simultaneous controlled demolition of the high molecular weight structure. Thus, it would appear that, as in HDS, such microbial treatment is accompanied by a loss in fuel value arising from the destruction of high molecular weight combustible hydrocarbons.

U.S. Patent No. 4,632,906, issued Dec. 30, 1986 (Kopacz, inv.; Atlantic Richfield Company, assignee) discloses a desulfurizing microorganism, *Bacillus sulfasportar*e ATCC No. 39909, said to react with organic sulfur associated with carbonaceous materials, e.g., coal or hydrocarbon oil. Kopacz theorizes that this microorganism removes sulfur by oxidation, and cleaves sulfur from its hydrocarbon matrix without the oxidation of carbon. Thus, the disclosed organism may accomplish certain objectives sought in the art for a microbial replacement for hydrodesulfurization.

Methods for reducing or stabilizing the viscosity of petroleum liquids without the need to expose these valuable fuel sources to extreme conditions are highly desirable. Chemical alteration of petroleum liquids under mild or atmospheric conditions has long been investigated. Some such chemical alterations occur naturally, for example those produced by microbial organisms which encounter and interact with the petroleum liquids. However, these chemical changes are usually deleterious to the fuel value of the petroleum liquids. For example, the viscosity of jet fuels increases upon storage over seawater, a customary practice in military installations. This is due to the formation of a sludge composed of bacteria which have attacked the fuel as a source of such nutrients as carbon, nitrogen, and sulfur. Upon physical agitation, the sludge becomes dispersed within the fuel liquid.

One possible method for preventing the accumulation of this sludge is proposed in U.S. Patent No. 3,069,325 (1962), by D.O. Hitzmann. This method is based upon the pretreatment of jet fuels with strains of bacteria adapted to the consumption of nitrogen- or sulfur-containing hydrocarbon fuel molecules as nutrient sources. When the nutrient-depleted fuel is thereafter stored over seawater, naturally occurring bacteria are unable to attack it as a metabolic substrate. Viscosity remains stable upon storage, but this result is achieved through the loss of combustible sulfur- and nitrogen-containing hydrocarbons.

Other microbial organisms have been described which produce substances conducive to the reduction of viscosity. Bertrand and coworkers, for example, describe one such microbial culture, of bacteria grown in seawater in the presence of crude oil. The culture produces tensioactive compounds, also known as biosurfactants, which assist in the dispersal of crude oil in seawater, thus facilitating the bioremediation of oil spills and chronic petroleum pollution. Bertrand et al., (1983) Biotechnology Letters 5(8):567-572. However, the culture also attacks and metabolizes hydrocarbon constituents of the petroleum, making it an unfavorable candidate for reducing the viscosity of valuable fuel liquids.

Although tensioactive compounds can be added during the manufacture of fossil fuels, see Scheffee, R.S. (1985) U.S. Patent No. 4,498,906, and may facilitate the efficiency of combustion of these fuels, the use of purified chemicals remains a less attractive viscosity-controlling alternative than harnessing microbial metabolic processes, which do not require the use of expensive additives. Moreover, any surfactant additives must be recovered from the petroleum during refining, which greatly complicates the refining process.

### SUMMARY OF THE INVENTION

This invention concerns the reduction of the viscosity of a liquid which contains sulfur aromatic heterocycles. These molecules must be present in sufficient relative abundance for their physicochemical properties to contribute significantly to the viscosity of the liquid. The present invention relates to the use of a biocatalytic agent comprising one or more microbial organisms expressing an enzyme that selectively cleaves at least one organic carbon-sulfur bond in aromatic heterocyclic molecules containing a sulfur heteroatom, or one or more enzymes derived from such microbial organisms, or mixtures of such micro-organisms and enzymes, to reduce the viscosity of a liquid which contains said molecules, the physicochemical properties of said molecules contributing significantly to the viscosity of said liquid.

The instant invention is particularly suitable for reducing the viscosity of petroleum liquids, such as crude oils or fractions thereof. This is due both to the alleviation of the need to expose the petroleum liquid to extreme conditions, such as those encountered in HDS, and to the unique, sulfur-specific nature of the biocatalysts employed. These biocatalytic agents possess the ability to selectively cleave carbon-sulfur bonds, and do not appreciably attack carbon-carbon bonds. Therefore, the fuel value of the petroleum liquid is not significantly diminished following biocatalytic treatment for the reduction of viscosity. The instant method can be used to reduce the viscosity of petroleum at numerous stages during its recovery and refining, including those steps at which other methods are impractical or impossible to employ.

### BRIEF DESCRIPTION OF DRAWINGS

Figure 1 depicts the structure of dibenzothiophene.

Figure 2 depicts the cleavage of dibenzothiophene by oxidative and reductive mechanisms, and the possible end products thereof.

Figure 3 depicts the stepwise oxidation of dibenzothiophene along the proposed "4S" pathway of microbial catabolism.

### DETAILED DESCRIPTION OF THE INVENTION

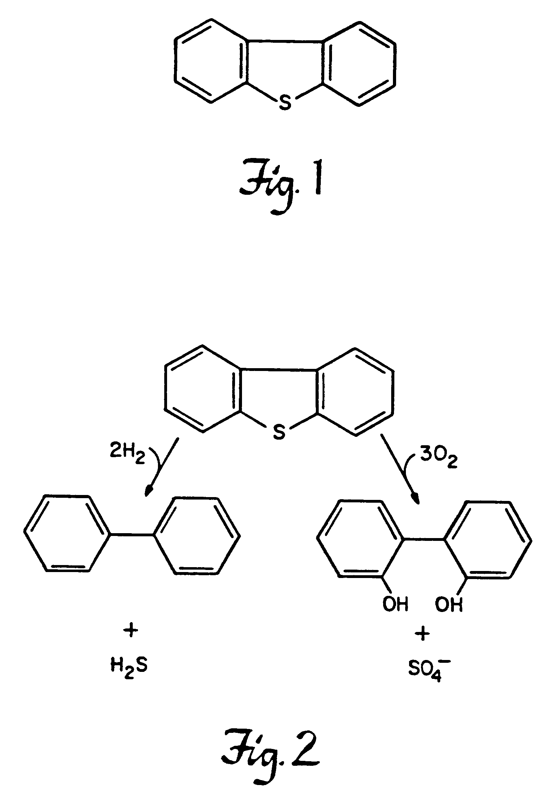

Aromatic heterocyclic molecules containing a sulfur heteroatom are herein referred to as sulfur heterocycles. These molecules often exist as rigid, planar condensed ring structures, which tend to form stable, organized liquids due to the strength of hydrophobic interactions between adjacent molecules. Such liquids are highly resistant to structural deformation by the application of external physical force, including elevated temperature and pressure. Thus, the presence of sulfur heterocycles can contribute to the observed viscous behavior of liquids. These conditions may be encountered in petroleum liquids generally, and are especially pronounced in high-sulfur petroleum such as Cerro Negro crude oil, which contains 4% organic sulfur. A sulfur heterocycle which illustrates these characteristics is dibenzothiophene (DBT), shown in Figure 1. DBT consists of a central thiophene ring flanked by two benzene ring moieties. DBT is representative of the class of sulfur heterocycles found in petroleum liquids; DBT itself may account for as much as 90% of the organic sulfur in some petroleum fractions.

The biocatalytic agents of the instant invention reduce the viscosity of liquids containing sulfur heterocycles, such as DBT, by converting these molecules into products which exhibit substantially different molecular characteristics than the parent heterocycles. Catalytic cleavage of carbon-sulfur bonds in sulfur heterocycles releases the overall structural rigidity of condensed ring molecules by breaking open the heterocyclic ring moieties, leaving single carbon-carbon bonds in their place. Figure 2 depicts two alternative biocatalytic mechanisms for accomplishing this result: reductive and oxidative sulfur-specific cleavage of heterocyclic ring moieties. In each case, freedom of rotation is acquired at the former heterocyclic ring sites; consequently, the liquid treated with the instant biocatalysts loses its high degree of molecular order and becomes more susceptible to structural deformation upon the application of external forces. Simple gravitational force or mildly elevated temperature or pressure will now be sufficient to induce flow.

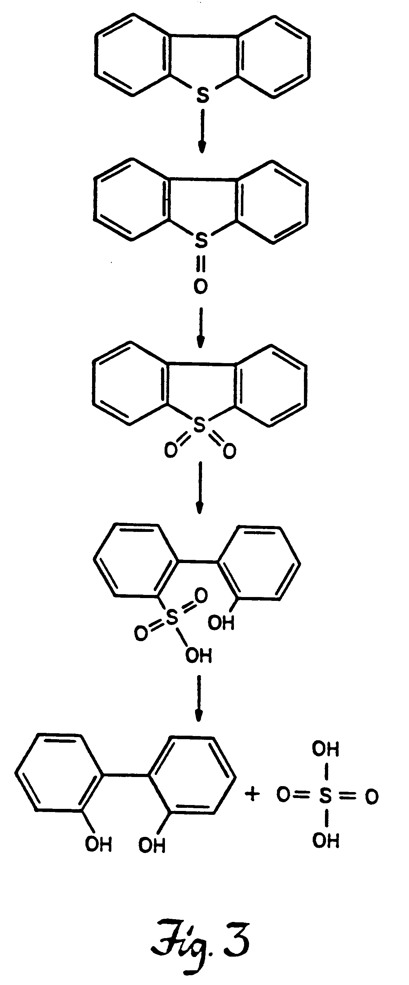

This result is preferentially accomplished using biocatalytic agents which selectively cleave organic carbon-sulfur bonds via the proposed "4S" pathway of oxidative catabolism, recently described by Kilbane, J.J., (1990) Resour. Conserv. Recycl. 3:69-79. The "4S" pathway is summarized in Figure 3. It consists of the sequential oxidation of the sulfur heteroatom, culminating in the release of inorganic sulfate from the aromatic hydrocarbon. The designation "4S" refers to the postulated sulfur intermediates: sulfoxide, sulfone, sulfonate, and the liberated product, inorganic sulfate. Ultimately, the "4S" pathway generates a hydrocarbon product which, in addition to cleavage of the heterocyclic ring moiety, flanking aromatic ring moieties may be hydroxylated. Acquisition of these polar ring substituents further decreases the importance of hydrophobic interactions between adjacent molecules; even lower viscosity will result.

It will be readily appreciated that for purposes of viscosity reduction, only the cleavage of a carbon-sulfur bond at the heterocyclic ring site is required. The liberation of inorganic sulfate is neither essential nor necessarily desirable for the purposes of the instant invention.

Naturally-occurring microbial organisms which carry out the sulfur-specific catabolism of sulfur heterocycles such as DBT have not yet been described. However, several researchers have disclosed the derivation of bacteria capable of selectively cleaving carbon-sulfur bonds from natural species, using metabolic selection combined with chemical mutagenesis. Isbister, J.D. and R.C. Doyle (1985) U.S. Patent No. 4,562,156 teaches one such method for the derivation of sulfur-specific bacteria from mixed cultures of the *Pseudomonas* species.

Similarly, Kilbane reports the derivation of a mixed bacterial culture capable of carrying out DBT catabolism via the "4S" pathway, using selective culture conditions coupled with chemical mutagenesis. Specifically, bacteria obtained from natural sources such as sewage sludge, petroleum refinery wastewater, garden soil, coal tar-contaminated soil, etc. are maintained in culture under conditions of continuous sulfur deprivation in the presence of sulfur heterocycles such as DBT. The culture is then exposed to the chemical mutagen 1-methyl-3-nitro-1-nitrosoguanidine. Kilbane reports the conversion of DBT into hydroxybiphenyl. Kilbane, J.J., (1990) Resour. Conserv. Recycl. 3:69-79.

Kilbane has further reported the isolation of a mutant strain of *Rhodococcus* rhodochrous bacteria from this mixed culture. This mutant, ATCC No. 53968, is a particularly preferred biocatalytic agent for use with the instant method for viscosity reduction. The isolation of this mutant is described in detail in J.J. Kilbane, U.S. Patent 5,104,801, issued on April 14, 1992 on an application filed January 5, 1990.

EP 0 445 896 discloses that ATCC No. 53968 and ATCC No. 53969 can be used to cleave carbon-sulphur bonds in the removal of organically bound sulphur from carbonaceous materials (page 13, lines 4-5). EP 0 445 896 mentions the treatment of solid, for example coal, or liquid materials.

In a preferred embodiment of the invention, an aqueous culture of ATCC No. 53968 is prepared by conventional fermentation under aerobic conditions, such as may be accomplished using a bioreactor and a suitable nutrient medium, comprising a conventional carbon source such as dextrose or glycerol. In order to generate maximal biocatalytic activity for the cleavage of organic carbon-sulfur bonds, it is important that the bacteria be maintained in a state of sulfur deprivation. Optionally, this may be accomplished using a medium lacking a source of inorganic sulfate, but supplemented with DBT or a crude oil or petroleum sample rich in sulfur heterocycles. When the culture has attained a sufficient volume and/or density, the crude oil, petroleum liquid or fraction thereof in need of biocatalytic treatment is contacted with it. The ratio of inoculum-to-substrate volumes may be varied widely, depending on the desired rate and degree of viscosity reduction; suitable ratios may be ascertained by those skilled in the art through no more than routine experimentation. Optimal ratios are those wherein the volume of inoculum used is up to one-tenth the total volume.

Any oxygen required for cleavage via the "4S" pathway can be supplied to the petroleum liquid in need of viscosity reduction prior to or during biocatalysis, using conventional techniques such as sparging or bubbling an oxygen source therethrough. Addition of the oxygen source directly to the petroleum liquid is preferable due to the greater solubility of oxygen in such liquids, relative to aqueous systems.

Mechanical agitation may also be desirable to accelerate the rate of viscosity reduction, but is not required. Suitable means for introducing mechanical agitation include, for example, incubation in a stirred-tank reactor.

Incubation temperatures in the range of about 10°C to 60°C are preferable, but any temperature between the pour point of the petroleum liquid and the temperature at which the biocatalytic agent is inactivated, can be used. Ambient temperature is particularly preferred.

Suitable incubation times for the desired degree of viscosity reduction will be readily ascertainable by those skilled in the art. Incubation with the biocatalyst need not be an isolated step, but may be conducted during the recovery, transit or storage of the substrate petroleum liquids.

Although the instant invention is concerned with the reduction in viscosity of crude oils, petroleum liquids and fractions thereof, those skilled in the art will recognize that it may facilitate the preparation of these liquids for desulfurization processes, in addition to generally converting them into forms which may be readily recovered, transported or refined using conventional techniques.

In other preferred embodiments of the instant invention, an enzyme or array of enzymes sufficient to direct the selective cleavage of organic Carbon-sulfur bonds are employed, rather than the intact microbial organisms expressing this enzymatic activity. For example, the enzyme or array of enzymes responsible for the "4S" series of reactions can be used to reduce the viscosity of petroleum liquids. The term "enzyme" herein refers to any protein biocatalyst possessing the functional characteristics desired. Thus, the "4S" enzyme or enzymes are defined as any enzyme which is capable of directing the catabolism of thiophene-containing sulfur heterocycles such as DBT through the "4S" series of reactions, including the enzymes expressed by ATCC No. 53968 and any functional derivatives thereof. These enzymes may optionally be used in carrier-bound form; suitable carriers include killed "4S" bacteria, fractions of said bacteria such as membrane fractions, insoluble resins, ceramic particles, latex particles and glass particles.

Optionally, the use of enzyme biocatalysts eliminates the need to prepare the biocatalytic agent in an aqueous medium; certain nonaqueous media such as perfluorochemicals, (PFCs) which are known to have a high capacity to dissolve oxygen, may be used to reconstitute or suspend the biocatalyst. A particularly useful PFC is perfluorobutyltetrahydrofuran, which is capable of dissolving a volume of oxygen equal to almost half the original volume of liquid, upon bubbling oxygen therethrough at room temperature. Lewis, R. (1990) Gen. Eng. News 10(5), at page 26. These oxygen-rich nonaqueous media may accelerate the rate of oxidative cleavage of carbon-sulfur bonds, by providing an oxygen-rich nonaqueous microenvironment, thus eliminating the formation of an emulsion between the aqueous biocatalyst and the hydrophobic petroleum liquid.

It will be appreciated that separation of the biocatalytic agents of the instant invention from the treated liquid is an optional step. Viscosity is reduced by converting sulfur heterocyclic components of the liquid to molecular forms which no longer confer viscous behavior. The continued presence of sulfur in either organic form (following cleavage of a single bond in the thiophene ring) or inorganic form (sulfate ions, in the case of "4S" oxidative cleavage) is of no consequence for the purposes of reducing viscosity. Moreover, as the biocatalytic agents of the instant invention do not appreciably cleave carbon-carbon bonds, there is no risk that continued presence of the biocatalyst will result in the destruction of the fuel value of petroleum treated by the method taught herein. In fact, the use of the instant method to reduce viscosity, rather than traditional methods such as HDS, confers the additional advantage of facilitating subsequent desulfurization of the treated petroleum.

Techniques for monitoring the progress of biocatalytic action are well-known and readily available to those skilled in the art. Suitable techniques include, but are not limited to, the collection of baseline and timecourse samples of the liquid being treated for direct analysis of viscosity in instruments such as a properly calibrated Saybolt viscometer. Alternatively, the disappearance of sulfur from aromatic hydrocarbons may be monitored using a gas chromatograph coupled with mass spectrophotometric detection (GC/MS), or with atomic emission spectral detection (flame spectrometry, GC/AES). Atomic emission detection allows the operator to visualize the disappearance of sulfur atoms from aromatic hydrocarbons by monitoring quantitative or relative decreases in emissions at 392 nm, the wavelength characteristic of atomic sulfur. The disappearance of sulfur can be correlated with decreasing viscosity, allowing the operator to choose either direct or indirect monitoring of biocatalysis once suitable calibration curves have been established.

This biocatalytic reduction of viscosity in crude oil, petroleum liquids or fractions thereof is suitable for use at many stages of the recovery, processing and refinement of petroleum. For example, the instant invention is well suited for use in facilitating the extraction of crude oils from the Earth. Preparations of the instant biocatalysts in an oxygen-enriched medium can be introduced to the oil while it is still within the Earth, as for example, by pumping the biocatalytic agent into a well or well shaft.

Alternatively, the biocatalyst can be contacted with the petroleum at the point of its extraction from the Earth, by use of a valve and mixing chamber at the well-head. The petroleum-biocatalyst mixture may then be pumped to a pipeline, storage reservoir or incubation chamber as desired.

The instant invention can also be used at any appropriate state in the transit, storage, or refining of the crude oils, petroleum liquids or fractions thereof, and may be used as a preliminary or companion step in other desirable manufacturing processes, such as desulfurization. Such manufacturing processes will be readily ascertainable by those skilled in the art.

There are many other potential uses for the viscosity reduction described herein, including the acceleration of bioremediation or biorecovery of petroleum pollutants released into the environment.

## Claims

1. Use of a biocatalytic agent comprising one or more microbial organisms expressing an enzyme that selectively cleaves at least one organic carbon-sulfur bond in aromatic heterocyclic molecules containing a sulfur heteroatom, or one or more enzymes derived from such microbial organisms, or mixtures of such micro-organisms and enzymes, to reduce the viscosity of a liquid which contains said molecules, the physicochemical properties of said molecules contributing significantly to the viscosity of said liquid.

2. The use of Claim 1, wherein the enzyme cleaves said at least one carbon-sulfur bond *via* stepwise oxidation, such that sulfoxide, sulfone, and sulfonate derivatives of the molecules are generated, and such that if at least two adjacent carbon-sulfur bonds are cleaved in a single molecule, inorganic sulfate is produced therefrom.

3. The use of Claim 1 or Claim 2, wherein the liquid containing said heterocyclic molecules is contacted with a source of oxygen, prior to or during incubation with the biocatalyst.

4. The use of Claim 1, 2 or 3, wherein the liquid containing said heterocyclic molecules is high sulfur, viscous petroleum.

5. The use of Claim 4, wherein the high sulfur, viscous petroleum is crude oil.

6. The use of any one of Claims 1 to 5, wherein the biocatalytic agent is a culture of a microbial organism expressing an enzyme that selectively cleaves at least one organic carbon-sulfur bond in said molecules, said culture being prepared under conditions of inorganic sulfur deprivation.

7. The use of any one of Claims 1 to 5, wherein the biocatalytic agent is a culture of Rhodococcus rhodochrous bacteria, ATCC No. 53968.

8. The use of Claim 1, wherein the enzyme is borne upon a carrier.

9. The use of any one of the preceding claims, wherein the carbon-sulfur bond cleavage occurs substantially without cleavage of the carbon-carbon bonds of the aromatic heterocyclic molecules.

## Patentansprüche

1. Verwendung eines biokatalytischen Agens, das eine oder mehrere mikrobielle Organismen enthält, die ein Enzym exprimieren, das selektiv zumindest eine organische Kohlenstoff-Schwefel-Bindung in aromatischen heterocyclischen Molekülen mit Schwefel als Heteroatom spaltet, oder ein oder mehrere Enzyme aus diesen mirobiellen Organismen oder Mischungen solcher Mikroorganismen und Enzymen enthält, zur Verminderung der Viskosität einer Flüssigkeit, die besagte Moleküle enthält, wobei deren physikalisch-chemische Eigenschaften zur Viskosität der besagten Flüssigkeit signifikant beitragen.

2. Die Verwendung gemäß Anspruch 1, wobei das Enzym die besagte, zumindest eine Kohlenstoff-Schwefel-Bindung durch schrittweise Oxidation so spaltet, daß Sulfoxid-, Sulfon-, und Sulfonat- Derivate der Moleküle gebildet werden und daß wenn zumindest zwei benachbarte Kohlenstoff-Schwefel-Bindungen in einem einzelnen Molekül gespaltet werden, anorganisches Sulfat daraus gebildet wird.

3. Die Verwendung gemäß Anspruch 1 oder Anspruch 2, wobei die Flüssigkeit, die die besagten heterocyclischen Moleküle enthält, mit einer Sauerstoffquelle vor oder während der Inkubation mit dem Biokatalysator in Kontakt gebracht wird.

4. Die Verwendung gemäß Anspruch 1, 2 oder Anspruch 3, wobei die Flüssigkeit, die die besagten heterocyclischen Moleküle enthält, viskoses Petroleum mit hohem Schwefelgehalt ist.

5. Die Verwendung gemäß Anspruch 4, wobei das viskose Petroleum mit hohem Schwefelgehalt Rohöl ist.

6. Die Verwendung gemäß einem der Ansprüche 1 bis 5, wobei das biokatalytische Agens eine Kultur eines mikrobiellen Organismus ist, der ein Enzym exprimiert, das selektiv zumindest eine organische Kohlenstoff-Schwefel-Bindung in dem besagten Molekülen spaltet, wobei die besagte Kultur unter Bedingungen des anorganischen Schwefel-Entzuges hergestellt ist.

7. Die Verwendung gemäß einem der Ansprüche 1 bis 5, wobei das biokatalytische Agens eine Kultur von Rhodococcus rhodochrous Bakterien ATCC Nummer 53968 ist.

8. Die Verwendung gemäß Anspruch 1, wobei das Enzym geträgert ist.

9. Die Verwendung gemäß einem der vorhergehenden Ansprüche, wobei die Spaltung der Kohlenstoff-Schwefel-Bindung im wesentlichen ohne Spaltung der Kohlenstoff-Kohlenstoff-Bindungen der aromatischen heterocyclischen Moleküle erfolgt.

## Revendications

1. Utilisation d'un agent biocatalytique comprenant un ou plusieurs organismes microbiens exprimant une enzyme qui coupe sélectivement au moins une liaison carbone-soufre organique dans des molécules hétérocycliques aromatiques contenant un hétéroatome de soufre, ou une ou plusieurs enzymes dérivées de tels organismes microbiens, ou des mélanges de tels microorganismes et enzymes, pour réduire la viscosité d'un liquide qui contient lesdites molécules, les propriétés physico-chimiques desdites molécules contribuant de façon significative à la viscosité dudit liquide.

2. Utilisation selon la revendication 1, dans laquelle l'enzyme coupe la ou lesdites liaison(s) carbone-soufre via une oxydation par étapes, telle que des dérivés sulfoxyde, sulfone et sulfonate des molécules sont produits, et telle que si au moins deux liaisons carbone-soufre adjacentes sont coupées dans une seule molécule, un sulfate inorganique est produit à partir de cette dernière.

3. Utilisation selon la revendication 1 ou la revendication 2 , dans laquelle le liquide contenant lesdites molécules hétérocycliques est mis en contact avec une source d'oxygène, avant ou pendant l'incubation avec le biocatalyseur.

4. Utilisation selon la revendication 1, 2 ou 3, dans laquelle le liquide contenant lesdites molécules hétérocycliques est du pétrole visqueux à haute teneur en soufre.

5. Utilisation selon la revendication 4, dans laquelle le pétrole visqueux à haute teneur en soufre est du pétrole brut.

6. Utilisation selon l'une quelconque des revendications 1 à 5, dans laquelle l'agent biocatalytique est une culture d'un organisme microbien exprimant une enzyme qui coupe sélectivement au moins une liaison carbone-soufre organique dans lesdites molécules, ladite culture étant préparée dans des conditions de carence en soufre inorganique.

7. Utilisation selon l'une quelconque des revendications 1 à 5, dans laquelle l'agent biocatalytique est une culture de bactérie Rhodococcus rhodochrous, déposée à l'ATCC sous le n° 53968.

8. Utilisation selon la revendication 1, dans laquelle l'enzyme est supportée sur un support.

9. Utilisation selon l'une quelconque des revendications précédentes, dans laquelle la coupure de la liaison carbone-soufre a lieu sensiblement sans coupure des liaisons carbone-carbone des molécules hétérocycliques aromatiques.
